# DEMANDE DE BREVET EUROPEEN

(11) **EP 3 319 059 A1**
(43) Date de publication de la demande: **09.05.2018**
(21) Numéro de dépôt: 17199692.9
(22) Date de dépôt: 02.11.2017
(51) Int. Cl.: G08B 21/04, A62B 35/00, G08B 25/01, G08B 25/08, G08B 29/20, G08B 29/18, G08B 25/00, A61B 5/11

(54) **PROCÉDÉ DE DÉTECTION D'UNE CHUTE D'UN TRAVAILLEUR ET DISPOSITIF DE DÉTECTION D'UNE CHUTE D'UN TRAVAILLEUR**

(30) Priorité: 03.11.2016 FR 1660614
(71) Demandeur: Fall - Alarmsystem, 38330 Montbonnot-sur-Martin (FR)
(72) Inventeur: CUNY, Arnaud, 38410 SAINT MARTIN D'URIAGE (FR)
(74) Mandataire: Lavoix

(57) **Abrégé**

Ce procédé de détection d'une chute d'un travailleur attaché à un dispositif d'ancrage comporte des étapes :
a) de mesure (102) d'une orientation d'un travailleur, au moyen d'un capteur d'orientation à six axes embarqué dans un dispositif de détection de chute solidarisé au travailleur ;
b) de calcul (104) de grandeurs statistiques représentatives de l'orientation mesurée ;
c) de comparaison (106) des grandeurs statistiques avec des valeurs de référence, une chute étant considérée comme détectée seulement si les grandeurs statistiques correspondent avec les valeurs de référence selon des critères prédéfinis, la comparaison étant réalisée au moyen d'un classifieur automatique.

## Description

La présente invention concerne un procédé de détection d'une chute avec dénivelé d'un travailleur. L'invention concerne également un dispositif de détection d'une chute avec dénivelé d'un travailleur.

On connaît des dispositifs de détection de chute, connus sous l'acronyme DATI, pour « dispositif d'alarme pour travailleur isolé » ou PTI, pour « protection du travailleur isolé », qui sont destinés à être portés par un travailleur et configurés pour détecter une perte de verticalité de ce travailleur. Lorsqu'une perte de verticalité est détectée, ces dispositifs sont configurés pour déclencher une alarme et contacter un centre d'assistance distant, de manière à porter secours au travailleur.

Ces dispositifs connus sont typiquement utilisés par des travailleurs devant travailler seuls, par exemple dans l'industrie, dans les métiers de la construction ou encore dans le transport de marchandises.

Ces dispositifs connus ont pour inconvénient qu'ils ne permettent pas de détecter une chute avec dénivelé, ni de discriminer entre une chute accidentelle, pour laquelle une assistance doit être apportée au travailleur, et une chute contrôlée, dans laquelle aucune assistance n'est nécessaire. Un exemple de chute contrôlée est lorsqu'un travailleur saute de son propre chef d'une faible hauteur, par exemple d'un marchepied ou d'une cabine de camion. Ainsi, ces dispositifs font courir le risque de ne pas détecter une chute véritable, alors qu'ils déclenchent inutilement des faux-positifs.

On connaît également les dispositifs décrits dans les documents US 2006/282021 A1, US 2012/119904 A1, US 2006/049950 A1 et FR 2785073 A1.

C'est à ces inconvénients qu'entend plus particulièrement remédier l'invention en proposant un procédé et un dispositif de détection de chute avec dénivelé, qui présente une fiabilité accrue, notamment en réduisant le risque de faux-positif.

A cet effet, l'invention concerne un procédé de détection d'une chute avec dénivelé d'un travailleur attaché à un dispositif d'ancrage conforme à la revendication 1.

Grâce à l'invention, la détection de chute de hauteur d'un travailleur est réalisée avec une fiabilité accrue, notamment lorsque ce travailleur est suspendu à un dispositif d'ancrage.

En effet, dans les dispositifs connus, la détection de la chute est basée sur une mesure de verticalité du corps du travailleur, le travailleur étant considéré comme ayant fait une chute en cas de perte de verticalité, c'est-à-dire lorsqu'il n'occupe plus une position verticale, par exemple parce qu'il est couché au sol.

Une telle détection n'est pas fiable lorsque le travailleur est accroché à un dispositif d'ancrage, car, dans ce cas, le travailleur peut conserver une position essentiellement verticale même après avoir fait une chute avec dénivelé.

En détectant les conditions de chute à partir des données de référence au moyen du classifieur, la probabilité de détecter une chute est plus élevée, même lorsque le travailleur n'est pas dans une position de perte de verticalité à l'issue de sa chute. De plus, cela permet de réduire les faux-positifs, c'est-à-dire la détection à tort de chutes lorsque le travailleur réalise un déplacement contrôlé tel qu'un saut.

Selon des aspects avantageux mais non obligatoires de l'invention, un tel procédé peut incorporer une ou plusieurs des caractéristiques des revendications dépendantes 2 à 12, prises isolément ou suivant toute combinaison techniquement admissible.

Selon un autre aspect, l'invention concerne un dispositif de détection d'une chute avec dénivelé d'un travailleur attaché à un dispositif d'ancrage, ce dispositif étant conforme à la revendication 13.

L'invention sera mieux comprise et d'autres avantages de celle-ci apparaîtront plus clairement à la lumière de la description qui va suivre, d'un mode de réalisation d'un procédé et d'un dispositif de détection de chute donné uniquement à titre d'exemple et faite en référence aux dessins annexés dans lesquels :
- la figure 1 est une représentation schématique d'un dispositif selon l'invention pour détecter une chute avec dénivelé d'un travailleur ;
- la figure 2 est une représentation schématique du dispositif de détection de la figure 1 ;
- la figure 3 est un ordinogramme d'un procédé de détection d'une chute de hauteur d'un travailleur conforme à l'invention ;
- la figure 4 est une représentation schématique d'un signal acquis par le dispositif de détection de la figure 1 lors de la chute d'un travailleur attaché à un dispositif d'ancrage.

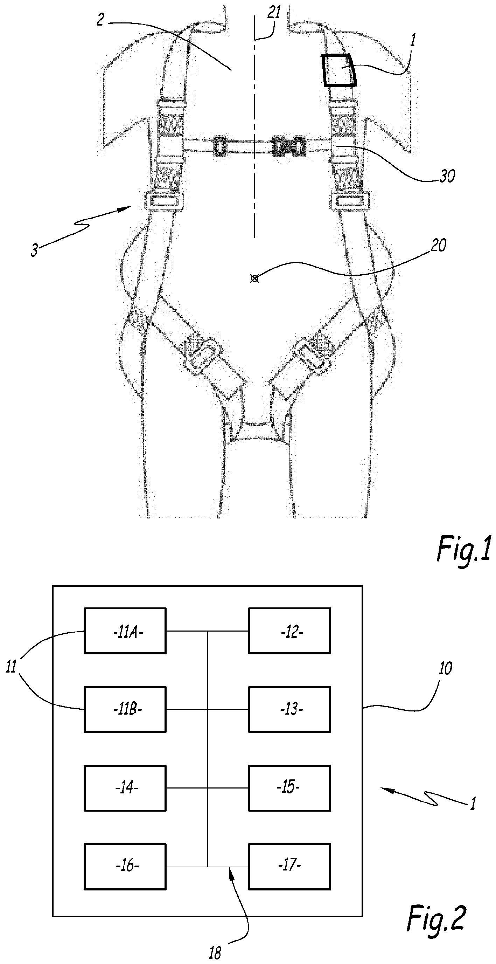

La figure 1 représente schématiquement un dispositif 1 de détection de chute d'un travailleur 2 humain.

Par exemple, le travailleur 2 est muni d'un harnais 3 de sécurité qui est accroché à un dispositif d'ancrage par l'intermédiaire d'un dispositif antichute, non illustré. L'accrochage du dispositif antichute au harnais de sécurité 3 peut être sternal ou dorsal, par exemple au moyen d'un mousqueton.

De façon connue, un dispositif d'ancrage peut être fixe ou mobile. Le dispositif antichute a pour fonction de retenir le travailleur 2 en cas de chute accidentelle, notamment lors d'une chute avec dénivelé, c'est-à-dire une chute en hauteur. Le dispositif antichute est par exemple une corde, un câble métallique ou une chaîne.

Le harnais de sécurité 3 comporte ici des sangles 30, par exemple en textile, qui sont disposées et serrées autour du corps du travailleur 2.

On note « 20 » le centre de gravité du travailleur 2 et « 21 » un axe longitudinal du travailleur 2. L'axe 21 est ici orienté selon une direction verticale lorsque le travailleur 2 se tient dans une position debout.

Le dispositif 1 est programmé pour détecter une chute accidentelle du travailleur 2. Le dispositif 1 est en outre programmé pour, lorsqu'une chute est détectée, déclencher une alarme comme expliqué dans ce qui suit.

Comme illustré à la figure 2, le dispositif 1 comporte un capteur d'orientation 11 à six axes, ou centrale inertielle, qui comporte un accéléromètre 11A trois axes et un gyroscope 11B à trois axes. Le capteur 11 est adapté pour mesurer une orientation du travailleur 2.

Le capteur 11 peut comporter l'accéléromètre 11A et le gyroscope 11B soit en tant que composants distincts, soit en tant qu'un même composant adapté pour implémenter ces deux fonctionnalités.

L'accéléromètre 11A est adapté pour mesurer un déplacement du travailleur 2, par exemple en mesurant une accélération du travailleur 2, selon trois axes géométriques orthogonaux d'un premier repère de référence. Ces axes de référence ne sont pas nécessairement alignés avec l'axe 21 du travailleur 2.

Le gyroscope 11B est adapté pour mesurer une position angulaire instantanée du travailleur 2 par rapport à trois axes géométriques orthogonaux d'un deuxième repère de référence. Par exemple, les premier et deuxième repères de référence correspondent à un même repère de référence du capteur 11.

Le capteur 11 est ainsi adapté pour mesurer, à chaque instant, trois composantes de position angulaire associées aux trois axes du premier repère et trois composantes d'accélération du travailleur 2 associées aux trois axes du deuxième repère. Le capteur 11 fournit, sur une interface de sortie, des signaux de mesure, représentatifs de ces mesures.

A titre illustratif, dans cet exemple, le capteur 11 est le capteur commercialisé par la société STMICROELECTRONICS sous la référence LSM6DS3.

L'utilisation d'un capteur à six-axes permet de mesurer l'orientation du travailleur 2 avec une meilleure précision et d'augmenter la fiabilité du procédé de détection d'une chute.

Le dispositif 1 comporte également une unité électronique de calcul programmable 12, une mémoire 13 et une interface homme-machine 14.

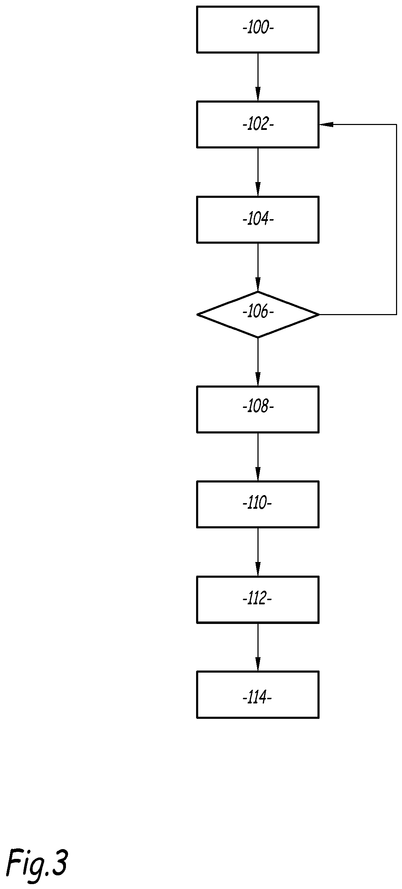

L'unité de calcul 12 est ici un microprocesseur ou un microcontrôleur programmable. L'unité 12 est notamment programmée pour exécuter des instructions stockées dans la mémoire 13 afin de mettre en oeuvre le procédé de détection d'une chute du travailleur 2 tel qu'illustré à la figure 3.

Par exemple, la mémoire 13 comporte un module de mémoire non volatile, ici de technologie Flash ou EEPROM.

L'interface homme-machine 14 est notamment adaptée pour afficher l'état du dispositif 1, par exemple l'état d'une liaison de données ou le niveau de charge d'une alimentation électrique ou encore pour afficher une alarme émise par le dispositif 1. L'interface homme-machine 14 est également adaptée pour permettre au travailleur 2 d'intervenir lors de l'exécution du procédé de détection, par exemple pour interrompre un signal d'alarme en cas de détection d'une chute, comme expliqué dans ce qui suit.

Par exemple, l'interface homme-machine 14 comporte des indicateurs lumineux, tels que des diodes électroluminescentes, des boutons poussoirs et/ou des interrupteurs. En variante, l'interface homme-machine 14 comporte un écran tactile.

Le dispositif 1 comporte avantageusement une interface de télécommunications 17 ainsi qu'un microphone 15 et un haut-parleur 16.

L'interface de télécommunications 17 est adaptée pour établir une liaison de données sans fil avec un serveur informatique distant, par exemple par l'intermédiaire d'un réseau de téléphonie sans fil ou du réseau internet. Ici, l'interface de télécommunications 17 est compatible avec les technologies de téléphonie sans fil GSM, UMTS et LTE. Par exemple, l'interface de télécommunications 17 contient une antenne radio.

Avantageusement, l'interface 17 permet l'envoi de données mesurées par le capteur 11 vers le serveur informatique distant.

Le dispositif 1 est en outre avantageusement programmé pour établir une communication vocale entre le travailleur 2 et un opérateur distant en cas de déclenchement d'une alarme consécutive à la détection d'une chute, de manière à ce que le travailleur 2 puisse transmettre, par la voix, grâce au microphone 15, des informations concernant son état. L'opérateur distant peut également écouter ce qui se passe même si le travailleur 2 ne répond pas.

En variante, le microphone 15 et le haut-parleur 16 peuvent être remplacés par un connecteur audio coaxial, par exemple du type « audio jack » en langue anglaise. La communication vocale est alors réalisée au moyen d'un casque et d'un microphone portés par le travailleur 2 et raccordés au dispositif 1 par l'intermédiaire de ce connecteur.

Le haut-parleur 16 est également adapté pour émettre un signal sonore d'alarme audible par le travailleur 2.

Le dispositif 1 comporte également un bus 18 de données, connecté aux constituants du dispositif 1, ici au capteur 11, à l'unité 12, à la mémoire 13, à l'interface 14, au microphone 15, au haut-parleur 16 et à l'interface de télécommunications 17, pour permettre un échange de données entre ces constituants. Selon un exemple illustratif, le bus de données 18 est une liaison filaire, formée par des pistes électriquement conductrices ménagées sur un circuit imprimé.

Le dispositif 1 comporte en outre un boîtier 10, de préférence réalisé en matière rigide et résistant aux chocs, à l'intérieur duquel sont logés les autres constituants du dispositif 1. L'interface homme-machine 14 est au moins en partie accessible au travailleur 2 depuis l'extérieur du boîtier 10.

Dans cet exemple, le boîtier 10 est pourvu, sur une face extérieure, de moyens de fixation destinés à le solidariser à une des sangles 30, tel qu'un crochet ou une bande de matériau auto-agrippant, par exemple de type Velcro®, adapté pour être attaché à une surface complémentaire de la sangle 30 du harnais de sécurité 3.

Le dispositif 1 est destiné à être solidarisé au travailleur 2, de préférence au-dessus du centre de gravité 20. Dans cet exemple, le dispositif 1 est attaché fixement sur une sangle 30 du harnais de sécurité 3.

De façon avantageuse et optionnelle, le dispositif 1 est placé au niveau de la poitrine du travailleur 2. De cette manière, les mesures acquises par le capteur 11 sont plus représentatives de la position du travailleur 2, ce qui accroît la fiabilité de la détection. Cela permet également au travailleur 2, lorsqu'une communication distante est établie suite à une alerte, de communiquer plus facilement avec l'opérateur distant au moyen du microphone 15 et haut-parleur 16, ces derniers étant alors plus près de la tête du travailleur 2.

Le dispositif 1 comporte en outre un système d'alimentation électrique, non illustré, tel qu'une batterie d'accumulateurs électriques rechargeables, adapté pour alimenter électriquement le dispositif 1 lors de son fonctionnement.

Avantageusement, le dispositif 1 comporte un connecteur de données, par exemple de type USB, pour « Universal Serial Bus » en langue anglaise, qui est raccordé au bus de données 18 et qui est accessible depuis l'extérieur du boîtier 10. Le connecteur permet l'échange de données entre le dispositif 1 et une unité centrale, non illustrée, par exemple pour charger des données dans la mémoire 13.

Par exemple, des données d'identification relatives au travailleur 2 sont transmises à la mémoire 13 depuis l'unité centrale au moyen de ce connecteur. Cela est particulièrement utile lorsque plusieurs travailleurs 2 sont amenés à utiliser un même dispositif 1 en succession les uns après les autres. De cette manière, le dispositif 1 est connecté à l'unité centrale avant d'être affecté à un travailleur, pour mettre à jour les données d'identification.

De façon particulièrement avantageuse, le connecteur est adapté pour permettre le rechargement du système d'alimentation électrique lorsqu'il est connecté à l'unité centrale.

En variante, l'interface 17 est en outre avantageusement configurée pour établir une liaison d'échange de données à courte portée, par exemple de type Bluetooth®, pour échanger de telles données avec l'unité centrale.

De façon optionnelle, le dispositif 1 comporte en outre un module de géolocalisation, ici connecté au bus de données 18. Par exemple, ce module de géolocalisation est adapté pour fonctionner avec un système de géolocalisation de type GPS, pour « Global Positioning System » en langue anglaise.

Dans cet exemple illustratif, le dispositif 1 est un appareil électronique dédié à la fonction de détection de chute.

En variante, le dispositif 1 est un terminal de communication mobile générique, par exemple de type tablette ou de type smartphone, qui est programmé pour implémenter les fonctions de détection de chute. Par exemple, une application exécutable dédiée est téléchargée, depuis un serveur informatique distant ou à partir d'un support amovible d'enregistrement d'informations tel qu'une carte mémoire, puis est installée dans la mémoire 13, de manière à pouvoir être exécutée par un système d'exploitation du dispositif de communication.

L'unité de calcul 12 est notamment programmée pour acquérir les valeurs d'orientation mesurées par le capteur d'inclinaison 11 et pour calculer des grandeurs statistiques représentatives des valeurs d'orientation mesurées.

Par exemple, les grandeurs statistiques sont choisies, pour chaque composante axiale du déplacement et de la position angulaire mesurées par le capteur 11, parmi l'ensemble composé :
- de la valeur minimale ;
- de la valeur maximale ;
- de la différence entre la valeur maximale et la valeur minimale ;
- du maximum de la valeur absolue de la différence première, la différence première étant définie comme égale à la différence entre deux valeurs consécutives de cette composante ;
- de l'écart entre le maximum et le minimum de la différence première ;
- de la variance ;
- de l'asymétrie, ou « skewness » en langue anglaise ;
- du kurtosis ;
- de la synchrovariance, définie comme la différence entre la plus grande valeur de variance et la plus petite valeur de variance quel que soit l'axe géométrique ;
- et de la différence entre la plus grande valeur et la plus petite valeur du maximum de la valeur absolue de la différence première quel que soit l'axe géométrique.

De préférence, au moins cinq des grandeurs statistiques ci-dessus sont utilisées, de préférence encore, l'ensemble des grandeurs statistiques ci-dessus sont utilisées.

Par exemple, ces grandeurs statistiques sont calculées par le calculateur 12 pour chacune des trois composantes axiales de position angulaire associées aux trois axes du premier repère et des trois composantes axiales d'accélération associées aux trois axes du deuxième repère, à partir des valeurs correspondantes mesurées, continûment ou de façon répétée, pendant une durée prédéfinie.

De préférence, ici, les mesures sont réalisées pendant une plage de temps prédéfinie, par exemple de durée égale à 1 seconde. Les grandeurs statistiques représentatives sont ensuite calculées à l'issue de cette plage de temps, à partir des valeurs mesurées pendant cette plage de temps prédéfinie. Par exemple, les valeurs mesurées par le capteur 11 pour des instants successifs sont stockées au fur et à mesure de leur acquisition dans un premier espace dédié de la mémoire 13 jusqu'à la fin de la plage de temps.

En outre, chaque plage de temps est ici partagée en plusieurs intervalles de temps prédéfinis, ici chacun de durée égale à 250ms. A l'issue de chaque intervalle, une nouvelle plage de temps est initiée, en parallèle de la plage de temps existante.

Par exemple, les valeurs mesurées par le capteur 11 pour des instants successifs sont stockées au fur et à mesure de leur acquisition dans un deuxième espace dédié de la mémoire 13, distinct du premier espace, jusqu'à la fin de cette deuxième plage de temps. En parallèle, les valeurs mesurées continuent à être stockées dans le premier espace mémoire jusqu'à la fin de la précédente plage de temps.

Ainsi, le dispositif de détection 1 est adapté pour acquérir des mesures d'orientation du travailleur 2 et les analyser pour détecter l'occurrence d'une chute en parallèle pour plusieurs plages de temps qui se recoupent au moins partiellement deux à deux.

L'analyse des grandeurs statistiques calculées pour détecter une chute du travailleur 2 est ici réalisée par l'unité 12 au moyen d'un classifieur automatique, qui reçoit en entrée les grandeurs statistiques calculées. Ici, cette analyse est réalisée indépendamment pour chacune des plages de temps prédéfinies.

Dans cet exemple, le classifieur est basé sur la méthode de la forêt aléatoire, ou « random decision forest » en langue anglaise, comportant un ensemble d'arbres de décision.

Le classifieur est préalablement entraîné à partir de données de référence, comme expliqué dans ce qui suit. Les arbres de décision formant la forêt sont automatiquement construits à partir des données de référence pendant une phase d'apprentissage du classifieur.

Pour chaque plage de temps, les grandeurs statistiques calculées pour chacune des six composantes précédemment décrites sont stockées dans un vecteur d'entrée, qui est fourni en entrée du classifieur.

Les arbres de décision sont adaptés pour, en réponse à ce vecteur d'entrée, classer ce vecteur d'entrée dans l'une ou l'autre de deux classes prédéfinies, l'une associée à une suspicion de chute et l'autre associée à une suspicion d'absence de chute. Ce classement comporte la comparaison des grandeurs statistiques calculées avec des valeurs de référence.

La chute est dite détectée seulement si les grandeurs statistiques calculées correspondent aux valeurs de référence selon des critères prédéterminés.

Les valeurs de référence et les critères prédéterminés sont fonction des données de référence avec lesquelles le classifieur a précédemment été entraîné.

Dans cet exemple, les comparaisons sont réalisées par un algorithme de décision en fonction des arbres de décision, la chute étant dite détectée seulement si la majorité des arbres décisionnels de la forêt d'arbres a classé le vecteur d'entrée correspondant dans la classe associée à la suspicion de chute.

Par exemple, la forêt comporte vingt arbres décisionnels avec une profondeur maximale supérieure ou égale à cinq ou à dix et de préférence inférieure ou égale à 53.

L'entraînement du classifieur est ici réalisé à partir de données de référence acquises à partir du dispositif 1 utilisé dans des essais contrôlés, pour lesquels les grandeurs statistiques calculées sont associées avec certitude à une situation de chute ou, au contraire, d'absence de chute.

Dans cet exemple, les essais ont été réalisés avec un mannequin représentatif d'un travailleur 2 et sur lequel le dispositif 1 est fixé de façon analogue à celle décrite ci-dessus. Ce mannequin est pourvu d'un harnais de sécurité 3 attaché à un dispositif d'ancrage. Le mannequin subit ensuite plusieurs chutes destinées à simuler des chutes réelles et pendant lesquelles le dispositif 1 enregistre, au moyen du capteur 11, l'orientation du mannequin de la même manière qu'il le ferait pour un travailleur 2. Plusieurs configurations de chute sont reproduites, en modifiant à chaque fois un ou plusieurs paramètres de chute, par exemple en modifiant :
- le sens de la chute : vers l'avant, latérale, en arrière ;
- l'accrochage du harnais de sécurité 3 au dispositif antichute de façon ventrale ou dorsale ;
- la nature du dispositif antichute et son état mécanique au moment de la chute, par exemple la longueur libre ou la tension mécanique ;
- les composants mécaniques utilisés pour relier le dispositif antichute, au harnais de sécurité 3 et au dispositif d'ancrage : longe simple ou double, présence ou non d'un absorbeur d'énergie, antichute sur support d'assurage, antichute à rappel automatique.

De préférence, plusieurs chutes, par exemple cinq, sont réalisées pour chaque combinaison.

En outre, plusieurs simulations de chutes contrôlées sont réalisées, par exemple en équipant un travailleur 2 du dispositif 1 et en le faisant réaliser des chutes contrôlées, par exemple sauter d'un marchepied ou d'une marche. Les mesures du capteur 11 sont alors enregistrées et les grandeurs statistiques associées sont calculées.

Le classifieur est alors entraîné à partir de ces données de référence.

Un exemple de mise en oeuvre d'un procédé de détection de chute du travailleur 2 conforme à l'invention est maintenant décrit en référence à l'ordinogramme de la figure 3.

De préférence, lors d'une étape préalable 100, un classifieur préalablement entraîné est acquis par le dispositif 1. Par exemple, les valeurs de référence et les règles prédéfinies correspondantes sont acquises et stockées dans la mémoire 13.

Ensuite, lors d'une étape 102, l'orientation du travailleur 2 est mesurée au moyen du capteur 11. Cette mesure est réalisée de façon continue ou pour des instants successifs au cours du temps pendant la plage de temps. Par exemple, comme indiqué précédemment, les mesures issues du capteur 11 sont utilisées pour plusieurs plages de temps distinctes et se recoupant entre elles deux à deux.

Ensuite, lors d'une étape 104, tout ou partie des grandeurs statistiques précédemment décrites représentatives des valeurs d'orientation mesurées sont calculées par l'unité électronique de calcul 12. Ce calcul est réalisé indépendamment à la fin de chaque plage de temps, à partir des valeurs mesurées pendant cette plage de temps.

Puis, lors d'une étape 106, les grandeurs statistiques calculées sont comparées avec des valeurs de référence prédéfinies. La comparaison est réalisée au moyen du classifieur automatique précédemment décrit et au moyen de l'unité électronique de calcul 12. Une chute étant considérée comme détectée seulement si les grandeurs statistiques correspondent avec les valeurs de référence. Dans le cas contraire, aucune chute n'est considérée détectée pour cette plage de temps.

Lorsqu'aucune chute n'est détectée à l'issue de l'étape 106, l'unité de calcul 12 analyse automatiquement les valeurs mesurées lors de la plage de temps suivante. L'étape 106 est ainsi mise en oeuvre successivement pour chaque plage de temps à l'issue de chaque mise en oeuvre de l'étape 104.

Grâce à l'invention, la détection de chute d'un travailleur en hauteur est réalisée avec une fiabilité accrue, notamment lorsque ce travailleur est suspendu à un dispositif d'ancrage.

En effet, dans les dispositifs connus, la détection de la chute est basée sur une mesure de verticalité du corps du travailleur, ce qui n'est pas fiable dans le cas où le travailleur 2 conserve une position essentiellement verticale même après avoir fait une chute.

En particulier, des essais expérimentaux ont montré qu'à l'issue d'une telle chute et lorsque le travailleur 2 est attaché au dispositif d'ancrage, l'axe 21 présente un angle par rapport à la verticale qui est inférieur ou égal à 60°, de préférence inférieur ou égal à 56°.

Avantageusement, lorsqu'une chute est détectée à l'issue de l'étape 106, alors, lors d'une étape 108, une demande de confirmation est adressée au travailleur 2. Cette demande comporte l'émission d'un signal par le dispositif 1 à destination du travailleur 2, par exemple un signal sonore émis au moyen du haut-parleur 16 et/ou un signal optique émis sur l'interface homme-machine 14. Cette demande de confirmation comporte également le déclenchement d'un décompte du temps qui s'écoule par l'unité de calcul 12.

La chute détectée est considérée comme confirmée si aucun signal d'acquittement du travailleur 2 n'est reçu par l'interface homme-machine 14 dans un délai prédéfini, par exemple à compter du moment où la chute est détectée. Le signal d'acquittement est par exemple envoyé par le travailleur 2 par pression d'un bouton de l'interface homme-machine 14.

Dans le cas contraire, si un signal d'acquittement du travailleur 2 est reçu par l'interface homme-machine 14 dans le délai prédéfini, alors la chute détectée est considérée comme étant un faux-positif et n'est pas prise en compte. Le dispositif 1 continue alors à exécuter les étapes 102, 104 et 106 sur les plages de temps suivantes.

Les étapes 102, 104 et 106 continuent à être mises en oeuvre pour des plages de temps successives par exemple jusqu'à l'arrêt du dispositif 1 ou jusqu'à ce qu'une chute soit détectée en fin d'étape 106 ou, avantageusement, après confirmation lors de l'étape 108.

De façon avantageuse, si une chute est confirmée à l'issue de l'étape 108, alors le dispositif 1 déclenche automatiquement une alarme. Ce déclenchement comporte par exemple l'émission d'un signal d'alerte vers un serveur informatique distant, au moyen de l'interface de télécommunications 17. Ce déclenchement peut également comporter l'émission d'un signal sonore par le haut-parleur 16.

Lorsque l'étape 108 est omise, l'étape 110 peut aussi être directement mise en oeuvre si une chute est détectée à l'issue de l'étape 106.

Puis, optionnellement, lors d'une étape 112, une communication vocale est établie entre le dispositif 1 et un opérateur distant. Par exemple, une communication téléphonique est établie au moyen de l'interface de télécommunications 17 vers un centre d'assistance dédié.

Cet appel téléphonique est ici initié par le dispositif 1, en contactant un numéro de téléphone prédéfini. En variante, l'appel téléphonique peut être initié par un serveur distant dès réception du signal d'alarme en sélectionnant un destinataire parmi une liste prédéfinie de contacts à joindre en cas d'urgence, en fonction de l'identifiant du travailleur 2.

De cette manière, le travailleur 2 peut, s'il est conscient, communiquer vocalement avec l'opérateur distant, à l'aide du microphone 15 et du haut-parleur 16, pour confirmer son état et, notamment, confirmer s'il a réellement fait une chute ou non. En fonction de la réponse du travailleur 2, l'opérateur distant commande l'envoi de secours ou, alternativement, commande l'arrêt de l'alarme si aucune chute n'a eu lieu.

De façon optionnelle, si la chute détectée est confirmée comme correspondant à une véritable chute, alors, lors d'une étape 114, les données mesurées par le capteur 11 sont envoyées automatiquement, par l'interface de télécommunications 17, à destination du serveur informatique distant. Par exemple, ces données sont utilisées pour mettre à jour la base de données de données de référence en vue de construire une nouvelle version de la forêt aléatoire.

De cette manière, le classifieur est mis à jour et enrichi par le serveur distant, en ajoutant aux données de référence des données qui sont associées à une chute considérée comme vérifiée.

De façon optionnelle, d'autres informations peuvent être transmises au serveur distant, par exemple grâce à l'interface 17, comme par exemple un identifiant du travailleur 2, la date et l'heure de la chute, du déclenchement de l'alarme et, le cas échéant de réception d'un signal d'acquittement.

Selon une variante, les coordonnées spatiales du travailleur 2 sont mesurées par le module de géolocalisation et la position correspondante est transmise automatiquement vers le serveur distant, par l'interface 17. Ce dernier est alors programmé pour afficher la position correspondante du travailleur 2 sur une carte. Ainsi, la position du travailleur 2 peut être suivie en temps réel. Cela est particulièrement avantageux lorsque le travailleur 2 est amené à évoluer dans un site industriel de taille importante.

Selon une autre variante, le dispositif 1 est adapté pour communiquer avec un dispositif 1 analogue. Les dispositifs 1 sont notamment programmés pour déclencher une alarme lorsqu'ils sont séparés l'un de l'autre par une distance supérieure à une distance prédéfinie. La distance est par exemple mesurée en temps réel par les unités de calcul 12 et à l'aide des modules de géolocalisation respectifs des deux dispositifs 1.

Cela est particulièrement avantageux pour éviter que deux travailleurs 2 devant travailler conjointement ne se séparent accidentellement après s'être perdu de vue, par exemple lorsqu'ils se déplacent dans un site industriel de taille importante, tel qu'une usine sidérurgique.

Cette dernière variante peut être mise en oeuvre indépendamment du procédé de détection de chute.

De façon générale, le dispositif 1 est plus particulièrement programmé pour détecter une chute accidentelle avec dénivelé du travailleur 2 lorsque ce travailleur 2 est attaché au dispositif d'ancrage, ce dernier empêchant le travailleur 2 de percuter le sol. Dit autrement, à l'issue de la chute, le travailleur 2 est suspendu en l'air par son dispositif antichute.

Par exemple, lorsque l'utilisateur 2 fait une chute avec dénivelé en étant attaché au dispositif d'ancrage, sa chute se décompose en plusieurs phases, au cours desquelles le travailleur 2 subit un mouvement différent de celui subi au cours des autres phases.

La chute commence par une première phase dite d'accélération, au cours de laquelle le travailleur 2 tombe en chute libre. Cette chute peut être accompagnée d'un mouvement de torsion du corps du travailleur 2.

Ensuite, lors d'une deuxième phase, la chute s'arrête brusquement, sous l'action du dispositif d'ancrage auquel le travailleur 2 est attaché, ici grâce au dispositif antichute et au harnais 3.

Enfin, lors d'une troisième phase, le travailleur 2 reste suspendu en l'air grâce au dispositif antichute, et subit des mouvements oscillants qui tendent à s'amortir avec le temps.

Une telle chute est donc différente d'une chute faite de plein pied par une personne marchant au sol. Dans une chute de plein pied, la personne subit un impact avec le sol. Dans le cas d'une chute avec dénivelé d'un travailleur attaché à un dispositif d'ancrage, le travailleur ne subit pas d'impact avec le sol. Il subit en revanche un mouvement d'arrêt brutal lorsque le dispositif antichute entre en action pour le retenir, mais ce mouvement est différent d'un impact avec le sol. Le travailleur 2 est arrêté en pleine chute sans toucher le sol. Les déplacements et accélérations subis par la travailleur 2 lors d'un arrêt en pleine chute, tels que mesurés par le ou les capteurs 11 du dispositif 1, sont différents de ceux subis lors d'une chute avec un impact sur le sol.

En d'autres termes, la chute du travailleur 2 présente une signature particulière.

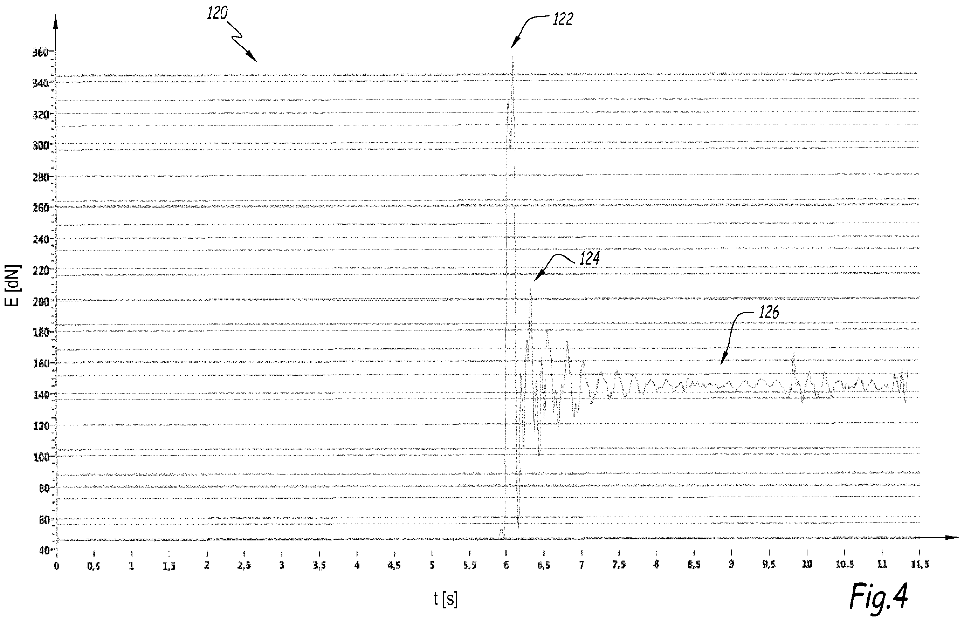

La figure 4 représente un exemple d'un signal 120 acquis par le dispositif 1 lorsque le travailleur 2 fait une chute avec dénivelé tout en étant attaché au dispositif d'ancrage, sans impact avec le sol.

Ce signal 120 est par exemple représentatif d'une accélération subie par le travailleur 2 mesurée à partir du capteur 11 suivant un des axes. Ce signal 120 est ici exprimé comme une force « E » exprimée en Newton, en fonction du temps « t » exprimé en secondes.

Le signal 120 comporte ici trois régions 122, 124 et 126 consécutives. Dans cet exemple illustratif, le travailleur 2 reste immobile préalablement à la chute.

La première région 122 correspond à la première phase précédemment décrite, c'est-à-dire à la chute libre. Elle comporte un ou plusieurs pics d'intensité élevée.

La deuxième région 124 correspond à la deuxième phase précédemment décrite, c'est-à-dire à l'arrêt de la chute. Elle comporte un ou plusieurs pics d'intensité sensiblement moins élevée que l'intensité des pics dans la première région 122.

La troisième région 126 correspond à la troisième phase précédemment décrite, c'est-à-dire au mouvement oscillant amorti du travailleur 2 suspendu au dispositif d'ancrage.

Ainsi, le dispositif 1 est avantageusement programmé, notamment au travers de l'unité électronique de calcul 12 et des instructions exécutables contenues dans la mémoire 13, pour détecter la chute à partir de l'orientation mesurée en tenant compte des différentes phases précédemment définies de la chute du travailleur 2.

Par exemple, lors de l'étape 104, les grandeurs statistiques sont calculées à partir d'une ou de plusieurs portions des signaux de mesure de l'orientation du travailleur 2 tels que mesurés par le capteur 11, chaque portion de signal étant représentative d'une phase de la chute du travailleur 2.

Par exemple, chaque composante axiale du déplacement et de la position angulaire mesurée se présente sous la forme d'un signal de mesure, ici délivré par le capteur 11. Pour chaque composante axiale du déplacement et de la position angulaire, c'est-à-dire pour chaque signal de mesure, les grandeurs statistiques choisies sont calculées pour plusieurs portions du signal correspondant et/ou pour plusieurs combinaisons de portions du signal correspondant, chacune de ces portions de signaux étant associée à une des phases de la chute telles que définies précédemment.

Avantageusement, le dispositif 1 est programmé pour identifier automatiquement, pour un signal de mesure donné, les portions correspondant à des phases de la chute par exemple sur la base d'une définition prédéfinie.

Le calcul des grandeurs statistiques choisies peut, par exemple, être fait séparément pour chaque portion d'un même signal de mesure, ou être réalisé pour plusieurs portions d'un même signal de mesure correspondant à des phases différentes de la chute du travailleur 2, ou être réalisé pour plusieurs portions de signaux de mesure différents mais correspondant à une même phase de la chute.

Lors de l'étape 106, la comparaison est réalisée en tenant compte des phases de la chute. Par exemple, les valeurs de références obtenues, ici par apprentissage, tiennent elles-mêmes compte de ces phases de la chute.

Les modes de réalisation et les variantes envisagés ci-dessus peuvent être combinés entre eux pour générer de nouveaux modes de réalisation.

## Revendications

1. Procédé de détection d'une chute d'un travailleur attaché à un dispositif d'ancrage, ce procédé étant **caractérisé en ce qu'**il comporte des étapes :
a) de mesure (102) d'une orientation d'un travailleur (2), au moyen d'un capteur d'orientation (11) à six axes adapté pour mesurer un déplacement suivant trois axes géométriques d'un premier repère de référence et pour mesurer une position angulaire par rapport à trois axes géométriques d'un deuxième repère de référence, ce capteur d'orientation (11) étant embarqué dans un dispositif (1) de détection de chute solidarisé au travailleur (2) ;
b) de calcul (104) de grandeurs statistiques représentatives de l'orientation mesurée, ce calcul étant réalisé par une unité électronique de calcul (12) du dispositif (1) de détection de chute ;
c) de comparaison (106) des grandeurs statistiques avec des valeurs de référence, une chute étant considérée comme détectée seulement si les grandeurs statistiques correspondent avec les valeurs de référence selon des critères prédéfinis, la comparaison étant réalisée au moyen d'un classifieur automatique mis en oeuvre par l'unité électronique de calcul (12).

2. Procédé de détection selon la revendication 1, **caractérisé en ce que**, les grandeurs statistiques calculées (104) lors de l'étape b) sont choisies, pour chaque composante axiale du déplacement et de la position angulaire mesurées par le capteur (11), parmi l'ensemble composé de la valeur minimale, de la valeur maximale, de la différence entre la valeur maximale et la valeur minimale, du maximum de la valeur absolue de la différence première, de l'écart entre le maximum et le minimum de la différence première, de la variance, de l'asymétrie, du kurtosis, de la synchrovariance définie comme la différence entre la plus grande valeur de variance et la plus petite valeur de variance quel que soit l'axe géométrique et de la différence entre la plus grande valeur et la plus petite valeur du maximum de la valeur absolue de la différence première quel que soit l'axe géométrique.

3. Procédé de détection selon l'une quelconque des revendications précédentes, **caractérisé en ce que** lors de l'étape c), le classifieur mis en oeuvre par l'unité de calcul (12) pour réaliser la comparaison (106) est une forêt aléatoire comprenant des arbres de décision, les arbres de décision ayant été préalablement construits à partir de données de référence lors d'une phase préalable d'apprentissage du classifieur, les valeurs de référence et les critères prédéfinis étant fonction des données de référence, les données de référence étant acquises à partir du dispositif de détection (1) lors d'essais contrôlés, pour lesquels les grandeurs statistiques calculées sont associées avec certitude à une situation de chute ou, au contraire, d'absence de chute.

4. Procédé de détection selon l'une quelconque des revendications précédentes, **caractérisé en ce que** le capteur d'orientation (11) comporte un accéléromètre trois-axes (11A) et un gyroscope (11B), et **en ce que**, lors de l'étape a), le déplacement suivant les trois axes géométriques est mesuré par l'accéléromètre (11A) et la position angulaire par rapport à ces trois axes géométriques est mesurée par le gyroscope (11B).

5. Procédé de détection selon l'une quelconque des revendications précédentes, **caractérisé en ce que**, pendant l'étape a), le capteur d'orientation (11) du dispositif de détection (1) est maintenu attaché au travailleur (2) en étant placé au-dessus du centre de gravité (20) du travailleur (2).

6. Procédé de détection selon l'une quelconque des revendications précédentes, **caractérisé en ce que**, si une chute est détectée à l'issue de l'étape c), alors le procédé comporte en outre une étape :
d) de demande de confirmation (108) au travailleur (2), comportant l'émission d'un signal par le dispositif (1) à destination du travailleur (2), la chute détectée étant considérée comme confirmée si aucun signal d'acquittement du travailleur (2) n'est reçu par une interface homme-machine (14) du dispositif (1) dans un délai prédéfini, la chute détectée étant considérée comme étant un faux-positif si un signal d'acquittement du travailleur (2) est reçu par l'interface homme-machine (14) dans le délai prédéfini.

7. Procédé de détection selon l'une quelconque des revendications précédentes, **caractérisé en ce qu'**il comporte une étape de déclenchement (110), par le dispositif de détection (1), d'une alarme lorsqu'une chute est détectée.

8. Procédé de détection selon l'une quelconque des revendications précédentes, **caractérisé en ce qu'**il comporte une étape de transmission (114) des grandeurs statistiques calculées à destination d'un serveur informatique distant lorsqu'une chute est détectée, cette transmission étant réalisée à l'aide d'une interface de télécommunications (17) du dispositif de détection (1).

9. Procédé de détection selon l'une quelconque des revendications précédentes, **caractérisé en ce que**, lors de l'étape a), des valeurs d'orientation sont mesurées (102) pendant une plage de temps prédéfinie, **en ce que** lors de l'étape b), les grandeurs statistiques représentatives sont calculées (104) à partir des valeurs mesurées pendant la plage de temps prédéfinie, et **en ce que** les étapes a), b) et c) sont exécutées par le dispositif de détection (1) pour plusieurs plages de temps prédéfinies qui se recoupent au moins partiellement deux à deux.

10. Procédé de détection selon l'une quelconque des revendications précédentes, **caractérisé en ce que** la chute du travailleur (2) est une chute avec dénivelé dans laquelle le travailleur (2) est empêché de percuter le sol grâce au dispositif d'ancrage.

11. Procédé de détection selon l'une quelconque des revendications précédentes, **caractérisé en ce que**, lors de l'étape b), les grandeurs statistiques sont calculées (104) à partir d'une ou de plusieurs portions des signaux de mesure de l'orientation du travailleur (2) tels que mesurés par le capteur de déplacement (11), chaque portion de signal étant représentative d'une phase de la chute du travailleur (2).

12. Procédé de détection selon la revendication 2, **caractérisé en ce que**, lors de l'étape b), chaque composante axiale du déplacement et de la position angulaire mesurée se présente sous la forme d'un signal de mesure et **en ce que**, pour chaque composante axiale du déplacement et de la position angulaire, c'est-à-dire pour chaque signal de mesure, les grandeurs statistiques choisies sont calculées pour plusieurs portions du signal correspondant et/ou pour plusieurs combinaisons de portions du signal correspondant, chacune de ces portions de signaux étant associée à une des phases de la chute.

13. Dispositif (1) de détection d'une chute d'un travailleur (2) attaché à un dispositif d'ancrage, ce dispositif étant destiné à être solidarisé au travailleur (2) et comportant :
- un capteur de déplacement (11) à six axes adapté pour mesurer un déplacement suivant trois axes géométriques d'un premier repère de référence et pour mesurer une position angulaire par rapport à trois axes géométriques d'un deuxième repère de référence ;
- une unité de calcul (12) électronique programmable ;
ce dispositif (1) étant **caractérisé en ce que** l'unité de calcul (12) est programmée pour mettre en oeuvre des étapes :
a) de mesure (102) d'une orientation du travailleur (2), au moyen du capteur de déplacement (11) ;
b) de calcul (104) de grandeurs statistiques représentatives des valeurs d'orientation mesurées ;
c) de comparaison (106) des grandeurs statistiques avec des valeurs de référence, une chute étant considérée comme détectée seulement si les grandeurs statistiques correspondent avec les intervalles de valeurs de référence selon des critères prédéfinis, la comparaison étant réalisée au moyen d'un classifieur automatique.
